# EUROPEAN PATENT APPLICATION

(11) **EP 3 892 605 A1**
(43) Date of publication of application: **13.10.2021**
(21) Application number: 19893399.6
(22) Date of filing: 03.12.2019
(51) Int. Cl.: C07C 1/04, C07C 11/02, C07C 11/06, C07C 15/02, C10G 2/00, B01J 29/072, B01J 35/04, C07B 61/00

(54) **PRODUCTION DEVICE FOR HYDROCARBONS AND PRODUCTION METHOD FOR HYDROCARBONS**

(30) Priority: 03.12.2018 JP 2018226927
(71) Applicant: Furukawa Electric Co., Ltd., Tokyo 100-8322 (JP)
(72) Inventor: NAKAI Yukako, Tokyo 100-8322 (JP); BANBA Yuichiro, Tokyo 100-8322 (JP); SEKINE Kaori, Tokyo 100-8322 (JP); NISHII Mai, Tokyo 100-8322 (JP); FUKUSHIMA Masayuki, Tokyo 100-8322 (JP); KATO Sadahiro, Tokyo 100-8322 (JP); MASUDA Takao, Sapporo-shi, Hokkaido 060-0808 (JP); NAKASAKA Yuta, Sapporo-shi, Hokkaido 060-0808 (JP); YOSHIKAWA Takuya, Sapporo-shi, Hokkaido 060-0808 (JP)
(74) Representative: Winter, Brandl - Partnerschaft mbB
(86) International application number: PCT/JP2019/047301
(87) International publication number: WO 2020/116478

(57) **Abstract**

Provided are an apparatus and method for producing hydrocarbons including aromatic hydrocarbons and/or lower olefins including propylene from CO H₂ while inhibiting reduction in catalyst activity and enhancing selectivity. The apparatus produces hydrocarbons including aromatic hydrocarbons having 6-10 carbon atoms and/or lower olefins including propylene, and is provided: a first supply unit which supplies a raw material gas containing CO and H₂; and a hydrocarbon production unit to which the raw material gas is supplied from the first supply unit, and which produces the hydrocarbons from CO and H₂ contained in the raw material gas while heating a catalyst structure at a temperature of 150°C or more and less than 300°C or at a temperature of 350°C or more and less than 550°C, the catalyst structure includes supports having a porous structure and including a zeolite-type compound, and a metal fine particle present in the supports, the supports have channels communicating with outside the supports, and a portion of the channels have an average inner diameter of 0.95 nm or less.

## Description

### TECHNICAL FIELD

The present invention relates to an apparatus and method for producing hydrocarbons.

### BACKGROUND ART

A known method of producing hydrocarbon compounds for use as raw materials for liquid or solid fuel products, such as synthetic fuels and synthetic oils as alternative fuels to petroleum, includes the Fischer-Tropsch synthesis reaction (hereinafter, also referred to as "FT synthesis reaction") by which hydrocarbons, specifically, liquid or solid hydrocarbons are catalytically produced from a synthesis gas composed mainly of carbon monoxide (CO) gas and hydrogen (H₂) gas.

Examples of the catalyst for use in the FT synthesis reaction include a catalyst disclosed in Patent Document 1, which includes a support, such as silica or alumina, and an active metal, such as cobalt or iron, on the support; and a catalyst disclosed in Patent Document 2, which includes cobalt, zirconium, or titanium, and silica.

The catalyst for use in the FT synthesis reaction can be obtained in the form of a supported cobalt or ruthenium oxide (unreduced catalyst) by, for example, impregnating a support, such as silica or alumina, with a cobalt or ruthenium salt and then calcining the impregnated support. The catalyst obtained in such a way should be made sufficiently active for the FT synthesis reaction. As disclosed in Patent Document 3, therefore, the obtained catalyst should be reduced by being brought into contact with a reducing gas, such as a hydrogen gas, so that the oxide of cobalt and/or ruthenium is converted into an active metal form.

During the FT synthesis reaction performed in a reactor, heavy hydrocarbons having a relatively large number of carbon atoms are produced mainly as liquid components while light hydrocarbons having a relatively small number of carbon atoms are produced mainly as gaseous components. Unfortunately, since hydrocarbons with various numbers of carbon atoms are synthesized during the FT synthesis reaction, the synthesis process needs to be followed by extra processes for obtaining desired hydrocarbons, such as cracking and purification processes. During the FT synthesis reaction, therefore, it is desirable to control as much as possible the distribution of the number of carbon atoms in the products.

Patent Document 4 discloses a core-shell catalyst including a core for serving as an FT synthesis catalyst; and a shell for cracking hydrocarbons to light ones, and discloses a technology using such a catalyst to allow the FT synthesis catalyst at the core to produce hydrocarbons and to allow the catalyst at the shell to crack the hydrocarbons, produced by the FT synthesis reaction, into light hydrocarbons. Unfortunately, Patent Document 4 only discloses the distribution of the number of carbon atoms in hydrocarbons mainly having five or more carbon atoms.

According to Patent Document 6, the number of carbon atoms in hydrocarbons produced at the core including a cobalt catalyst is controlled by the reaction at the surface of zeolite in the shell. Thus, even after the cracking of the products is controlled at the shell, the products may react again with the core or another adjacent shells. This makes it difficult to control the selectivity for hydrocarbons with the number of carbon atoms in a specific range, and specifically makes it difficult to control the distribution of the number of carbon atoms when light hydrocarbons with a small number of carbon atoms are to be produced.

Various aromatic hydrocarbons and light hydrocarbons, such as propylene and butene, produced as gaseous components during the FT synthesis reaction are known as basic raw materials for use in production of various chemicals. Although such compounds can be produced during the FT synthesis reaction, the selectivity for the production of such compounds still remains low. In other words, no techniques have been established for increasing the selectivity for such compounds produced by the FT synthesis reaction. A need also exists to develop such techniques not only for improving the compound selectivity but also for improving the production efficiency by omitting extra processes, such as purification, necessary after the synthesis process in the conventional art.
Patent Document 1: Japanese Unexamined Patent Application, Publication No. H04-227847
Patent Document 2: Japanese Unexamined Patent Application, Publication No. S59-102440
Patent Document 3: PCT International Publication No. WO2015/072573
Patent Document 4: PCT International Publication No. WO2014/142282

### DISCLOSURE OF THE INVENTION

### Problems to be Solved by the Invention

It is an object of the present invention to provide an apparatus and method for producing hydrocarbons, which make it possible to efficiently and highly selectively produce, from carbon monoxide and hydrogen, hydrocarbons including at least one compound selected from lower olefins including propylene and aromatic hydrocarbons with less decrease in catalytic activity.

### Means for Solving the Problems

The present invention has the following principal configuration.
1. An apparatus for producing hydrocarbons including at least one compound selected from lower olefins including propylene and aromatic hydrocarbons having six or more and ten or less carbon atoms, the apparatus including: a first supply unit that supplies a raw material gas including carbon monoxide and hydrogen; and a hydrocarbon production unit that includes a catalyst structure, receives supply of the raw material gas from the first supply unit, and produces, from carbon monoxide and hydrogen in the raw material gas, hydrocarbons including at least one compound selected from lower olefins including propylene and aromatic hydrocarbons having six or more and ten or less carbon atoms while heating the catalyst structure at a first temperature of 150°C or more and less than 300°C or at a second temperature of 350°C or more and less than 550°C, wherein the catalyst structure includes supports each having a porous structure and including a zeolite-type compound, and at least one metal fine particle present in the supports, the supports have channels communicating with outside the supports, and some of the channels have an average inner diameter of 0.95 nm or less.
2. The apparatus for producing hydrocarbons according to aspect 1, wherein the aromatic hydrocarbons include at least one of a polycyclic aromatic hydrocarbon and a compound represented by formula (1): 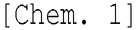 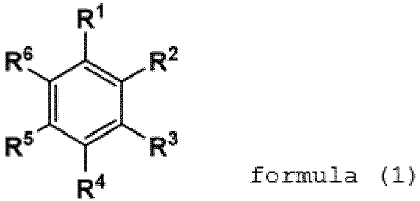 wherein R¹, R², R³, R⁴, R⁵, and R⁶ are each independently a hydrogen atom or an alkyl group having one or more and four or less carbon atoms.
3. The apparatus for producing hydrocarbons according to aspect 2, wherein the compound represented by formula (1) includes one or more compounds selected from the group consisting of benzene, toluene, and butylbenzene.
4. The apparatus for producing hydrocarbons according to aspect 2, wherein the polycyclic aromatic hydrocarbon includes at least one of azulene and naphthalene.
5. The apparatus for producing hydrocarbons according to any one of aspects 1 to 4, further including an oxygen supply unit that supplies oxygen to the hydrocarbon production unit.
6. The apparatus for producing hydrocarbons according to aspect 5, wherein the oxygen supply unit supplies, to the hydrocarbon production unit, oxygen at a concentration below an explosive limit.
7. The apparatus for producing hydrocarbons according to any one of aspects 1 to 6, wherein the metal fine particle includes at least one first metal selected from the group consisting of ruthenium (Ru), nickel (Ni), iron (Fe), and cobalt (Co) or includes at least one first metal selected from the group consisting of ruthenium (Ru), nickel (Ni), iron (Fe), and cobalt (Co) and at least one second metal being different from the first metal and selected from the group consisting of platinum (Pt), palladium (Pd), gold (Au), silver (Ag), copper (Cu), ruthenium (Ru), iridium (Ir), rhodium (Rh), osmium (Os), zirconium (Zr), and manganese (Mn).
8. The apparatus for producing hydrocarbons according to any one of aspects 1 to 7, wherein some of the channels have an average inner diameter of 0.39 nm or more and 0.75 nm or less.
9. The apparatus for producing hydrocarbons according to any one of aspects 1 to 8, wherein the channels have any one of a one-dimensional pore, a two-dimensional pore, and a three-dimensional pore of a framework structure of the zeolite-type compound, and have an enlarged pore portions different from the one-dimensional pore, the two-dimensional pore, and the three-dimensional pore, and the metal fine particle is present at least in the enlarged pore portion.
10. The apparatus for producing hydrocarbons according to any one of aspects 1 to 9, further including a first separation unit that separates hydrogen, carbon monoxide, and the hydrocarbons discharged from the hydrocarbon production unit.
11. The apparatus for producing hydrocarbons according to aspect 10, further including a second supply unit that supplies, to the hydrocarbon production unit, carbon monoxide and hydrogen separated by the first separation unit.
12. The apparatus for producing hydrocarbons according to any one of aspects 1 to 11, further including a detection unit that detects the propylene and the aromatic hydrocarbons in the hydrocarbons discharged from the hydrocarbon production unit.
13. The apparatus for producing hydrocarbons according to any one of aspects 1 to 12, further including a second separation unit that cools the hydrocarbons discharged from the hydrocarbon production unit and separates the lower olefins and the aromatic hydrocarbons.
14. A method for producing hydrocarbons including at least one compound selected from lower olefins including propylene and aromatic hydrocarbons having six or more and ten or less carbon atoms, the method including: a step S1 that includes supplying, to a catalyst structure, a raw material gas including carbon monoxide and hydrogen; and a step S2 that includes producing, from carbon monoxide and hydrogen in the raw material gas, hydrocarbons including at least one compound selected from lower olefins including propylene and aromatic hydrocarbons having six or more and ten or less carbon atoms while heating the catalyst structure at a first temperature of 150°C or more and less than 300°C or at a second temperature of 350°C or more and less than 550°C, wherein the catalyst structure includes supports each having a porous structure and including a zeolite-type compound, and at least one metal fine particle present in the supports, the supports have channels communicating with outside the supports, and some of the channels have an average inner diameter of 0.95 nm or less.

### Effects of the Invention

The present invention makes it possible to provide an apparatus and method for producing hydrocarbons, which make it possible to efficiently and highly selectively produce, from carbon monoxide and hydrogen, hydrocarbons including at least one compound selected from lower olefins including propylene and aromatic hydrocarbons with less decrease in catalytic activity.

### BRIEF DESCRIPTION OF THE DRAWINGS

FIG. 1 is a block diagram showing, as blocks, principal components of an apparatus for producing hydrocarbons according to an embodiment.
FIGS. 2(a) and 2(b) are views schematically showing the inner structure of a catalyst structure used in the apparatus for producing hydrocarbons, in which FIG. 2(a) is a perspective view (shown partially in transverse cross-sectional view), and FIG. 2(b) is a partially enlarged cross-sectional view.
FIG. 3 is a schematic view showing a modification of the catalyst structure of FIGS. 2(a) and 2(b).

### PREFERRED MODE FOR CARRYING OUT THE INVENTION

Hereinafter, embodiments will be described with reference to the drawings.

As a result of intensive studies for achieving the object, the inventors have completed the present invention based on the findings that the use of a certain catalyst structure makes it possible to efficiently produce hydrocarbons including at least one compound selected from lower olefins including propylene and aromatic hydrocarbons with improved selectivity and less decrease in catalytic activity.

The hydrocarbon production apparatus according to an embodiment is an apparatus for producing hydrocarbons including at least one compound selected from lower olefins including propylene and aromatic hydrocarbons having six or more and ten or less carbon atoms, the apparatus including: a first supply unit that supplies a raw material gas including carbon monoxide and hydrogen; and a hydrocarbon production unit that includes a catalyst structure, receives supply of the raw material gas from the first supply unit, and produces, from carbon monoxide and hydrogen in the raw material gas, hydrocarbons including at least one compound selected from lower olefins including propylene and aromatic hydrocarbons having six or more and ten or less carbon atoms while heating the catalyst structure at a first temperature of 150°C or more and less than 300°C or at a second temperature of 350°C or more and less than 550°C, in which the catalyst structure includes supports each having a porous structure and including a zeolite-type compound, and at least one metal fine particle present in the supports, the supports have channels communicating with outside the supports, and some of the channels have an average inner diameter of 0.95 nm or less.

The hydrocarbon production method according to an embodiment is a method for producing hydrocarbons including at least one compound selected from lower olefins including propylene and aromatic hydrocarbons having six or more and ten or less carbon atoms, the method including: a step S1 that includes supplying, to a catalyst structure, a raw material gas including carbon monoxide and hydrogen; and a step S2 that includes producing, from carbon monoxide and hydrogen in the raw material gas, hydrocarbons including at least one compound selected from lower olefins including propylene and aromatic hydrocarbons having six or more and ten or less carbon atoms while heating the catalyst structure at a first temperature of 150°C or more and less than 300°C or at a second temperature of 350°C or more and less than 550°C, in which the catalyst structure includes supports each having a porous structure and including a zeolite-type compound, and at least one metal fine particle present in the supports, the supports have channels communicating with outside the supports, and some of the channels have an average inner diameter of 0.95 nm or less.

### Configuration of Hydrocarbon Production Apparatus

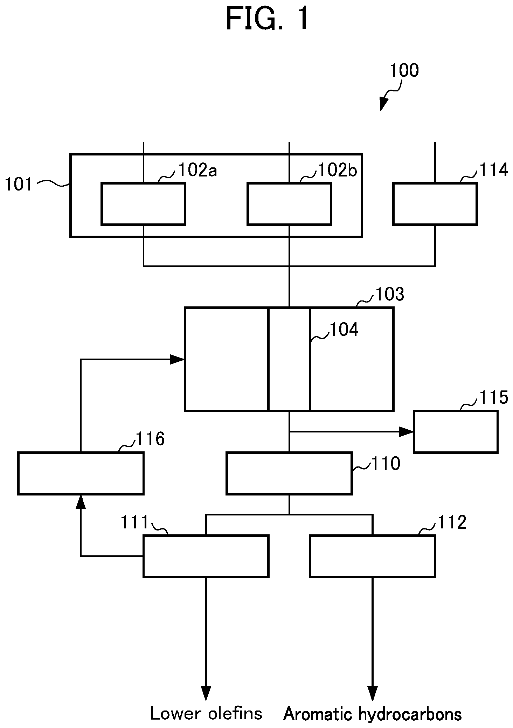

FIG. 1 is a block diagram showing, as blocks, principal components of the hydrocarbon production apparatus according to an embodiment of the present invention.

As shown in FIG. 1, the hydrocarbon production apparatus 100 (hereinafter also simply referred to as "production apparatus") is an apparatus for producing hydrocarbons including at least one compound selected from lower olefins including propylene and aromatic hydrocarbons having six or more and ten or less carbon atoms and includes a first supply unit 101 and a hydrocarbon production unit 103 as main components.

The first supply unit 101 includes a carbon monoxide supply unit 102a and a hydrogen supply unit 102b and supplies, to the hydrocarbon production unit 103, a raw material gas including carbon monoxide and hydrogen. The carbon monoxide supply unit 102a includes, for example, a carbon monoxide gas cylinder or a carbon monoxide gas generator and may be configured to control the supply of carbon monoxide gas to the hydrocarbon production unit 103. The hydrogen supply unit 102b includes, for example, a hydrogen gas cylinder or a hydrogen gas generator and may be configured to control the supply of hydrogen to the hydrocarbon production unit 103.

The hydrocarbon production unit 103 includes a catalyst structure holder 104 that holds a catalyst structure; and a heating unit (not shown) capable of controlling temperature, such as a heating furnace, and is configured to heat, at a first temperature of 150°C or more and less than 300°C or at a second temperature of 350°C or more and less than 550°C, the catalyst structure in the catalyst structure holder 104 by means of, for example, a heating furnace. The hydrocarbon production unit 103 receives supply of the raw material gas including carbon monoxide and hydrogen from the first supply unit 101, and produces, from carbon monoxide and hydrogen in the raw material gas, hydrocarbons including lower olefins including propylene and aromatic hydrocarbons having six or more and ten or less carbon atoms while heating, at a predetermined temperature, the catalyst structure in the catalyst structure holder 104.

Specifically, while a synthesis gas including carbon monoxide and hydrogen is supplied from the first supply unit 101 to the hydrocarbon production unit 103, the supplied carbon monoxide and hydrogen are allowed to react with the catalyst structure being heated at a first temperature of 150°C or more and less than 300°C or at a second temperature of 350°C or more and less than 550°C in the hydrocarbon production unit 103, so that hydrocarbons including lower olefins including propylene and aromatic hydrocarbons having 6 or more and ten or less carbon atoms are produced. Hereinafter, the hydrocarbon producing reaction occurring in the hydrocarbon production unit 103 is also referred to as FT synthesis reaction.

When the catalyst structure is heated at the first temperature, lower olefins can be efficiently produced by FT synthesis reaction. When the catalyst structure is heated at the second temperature, aromatic hydrocarbons can be efficiently produced by FT synthesis reaction.

In the hydrocarbon production unit 103, the catalyst structure holder 104 may be installed in any manner that allows contact between the raw material gas and the catalyst structure being heated in the catalyst structure holder 104. As shown in FIG. 1, for example, the catalyst structure holder 104 may be installed to connect the connecting portion between the piping for the raw material gas supply and the hydrocarbon production unit 103 to the connecting portion between the hydrocarbon production unit 103 and the hydrocarbon discharge piping.

The hydrocarbons produced by the production apparatus 100 include propylene and aromatic hydrocarbons. The concentrations of propylene and aromatic hydrocarbons in the hydrocarbons may be at any levels and selected as appropriate by controlling the conditions for the FT synthesis reaction.

Besides propylene, the lower olefins in the hydrocarbons may include olefins having four or less carbon atoms, such as ethylene, propylene, and butene.

The aromatic hydrocarbons in the hydrocarbons may include at least one of a polycyclic aromatic hydrocarbon and a compound represented by formula (1): 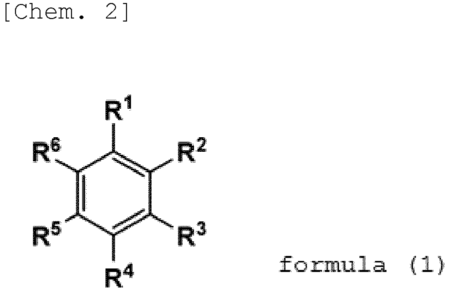 wherein R¹ to R⁶ are each independently a hydrogen atom or an alkyl group having one or more and four or less carbon atoms.

The compound represented by formula (1) may include one or more selected from the group consisting of benzene, toluene, and butylbenzene. The polycyclic aromatic hydrocarbon may include at least one of azulene and naphthalene.

The hydrogen and carbon monoxide supplied to the catalyst structure holder 104 preferably have a hydrogen/carbon monoxide molar ratio of 1.5 or more and 2.5 or less and more preferably 1.8 or more and 2.2 or less. The pressure (absolute pressure) in the catalyst structure holder 104 is preferably 0.1 MPa or more and 3.0 MPa or less. Under such conditions, the FT synthesis reaction can efficiently proceed in the hydrocarbon production unit 103.

The second temperature, which is the higher catalyst structure-heating temperature, is preferably 350°C or more and 550°C or less and more preferably 450°C or more and 550°C or less. When the catalyst structure is heated at a temperature in the above range, the aromatic hydrocarbons can be selectively produced, which results in an increase in the production of desired hydrocarbons.

The production apparatus 100 allows the catalyst structure to be heated at a predetermined temperature so that hydrocarbons including lower olefins including propylene and aromatic hydrocarbons having six or more and ten or less carbon atoms can be produced from the raw material gas including carbon monoxide and hydrogen. Therefore, the production apparatus 100 makes it possible to produce a desired type of hydrocarbons by adjusting the catalyst structure heating temperature without catalyst exchange.

The production apparatus 100 may further include an oxygen supply unit 114 that supplies oxygen to the hydrocarbon production unit 103. The oxygen supply unit 114 may include, for example, an oxygen gas cylinder or an oxygen gas generator and may be configured to control the supply of oxygen gas to the hydrocarbon production unit 103.

The supply of oxygen from the oxygen supply unit 114 to the catalyst structure in the catalyst structure holder 104 can prevent a decrease in the catalytic activity of the catalyst structure, which would otherwise be caused by FT synthesis reaction-induced coking of the catalyst structure.

The oxygen supply unit 114 preferably supplies, to the hydrocarbon production unit 103, oxygen at a concentration below the explosive limit. The supply of oxygen at such a concentration to the hydrocarbon production unit 103 can efficiently prevent a decrease in the catalytic activity of the catalyst structure, which would otherwise be caused by coking.

Specifically, a larger amount of oxygen supplied to the hydrocarbon production unit 103 can more effectively prevent the coking-induced deactivation of the catalyst structure, in which the amount of oxygen should have an upper limit that makes the oxygen concentration lower than the explosive limit. Oxygen supplied from the oxygen supply unit 114 may be mixed with inert gas, such as nitrogen or argon, so that the oxygen concentration is adjusted to a predetermined level.

The production apparatus 100 may further include a first separation unit 111 that separates hydrogen, carbon monoxide, and the hydrocarbons discharged from the hydrocarbon production unit 103. The first separation unit 111 is preferably, for example, a cryogenic separation unit that separates, from the hydrocarbons, carbon monoxide and hydrogen, which are raw material gas components remaining unreacted during the FT synthesis reaction in the hydrocarbon production unit 103. The separation of carbon monoxide and hydrogen from the hydrocarbons allows production of the hydrocarbons at a high volume content.

The production apparatus 100 may further include a second supply unit 116 that supplies, to the hydrocarbon production unit 103, carbon monoxide and hydrogen separated by the first separation unit 111. The second supply unit 116 may be configured to control the supply of carbon monoxide and hydrogen to the hydrocarbon production unit 103. The carbon monoxide and hydrogen supplied from the second supply unit 116 to the hydrocarbon production unit 103 can be reused for the FT synthesis reaction, which reduces the amount of the raw material gas used.

The production apparatus 100 may further include a detection unit 115 that detects propylene and aromatic hydrocarbons in the hydrocarbons produced in and discharged from the hydrocarbon production unit 103. When the detection unit 115 successfully detects propylene and the aromatic hydrocarbons in the hydrocarbons, the production apparatus 100 may be determined to be operating normally. When the detection unit 115 fails to detect propylene and aromatic hydrocarbons in the hydrocarbons, the production apparatus 100 may be determined to be out of order, and the part involved in the FT synthesis reaction should be deactivated and inspected or repaired.

The production apparatus 100 may further include a second separation unit 110 that cools the hydrocarbons discharged from the hydrocarbon production unit 103 and separates a lower olefin-containing gas and the aromatic hydrocarbons. The second separation unit 110 is, for example, a gas-liquid separator capable of cooling to room temperature. The lower olefin-containing gas may include lower olefins, carbon monoxide, and hydrogen.

The production apparatus 100 may further include a third separation unit 112 that separates water in the aromatic hydrocarbon fraction discharged from the second separation unit 110. The third separation unit 112 is, for example, a separator that performs fractional distillation according to boiling points. The water separated by the third separation unit 112 may be handled as industrial wastewater.

FIG. 1 shows an example in which carbon monoxide and hydrogen are supplied respectively by the carbon monoxide supply unit 102a and the hydrogen supply unit 102b. Alternatively, a gas of a mixture of carbon monoxide and hydrogen may be supplied to the hydrocarbon production unit 103. FIG. 1 also shows an example in which the raw material gas and oxygen are supplied respectively by the first supply unit 101 and the oxygen supply unit 114. Alternatively, a gas of a mixture of the raw material gas and oxygen may be supplied to the hydrocarbon production unit 103.

The carbon monoxide supply unit 102a may supply carbon monoxide at any concentration, and the hydrogen supply unit 102b may supply hydrogen at any concentration. For example, the concentrations of carbon monoxide and hydrogen may be 100% or adjusted to specific values by the mixing with inert gas, such as nitrogen or argon. When inert gas is used, an inert gas supply unit (not shown) may be provided to supply inert gas to the production apparatus 100.

FIG. 1 shows an example in which the catalyst structure is installed in the catalyst structure holder 104. The catalyst structure may be installed in any manner that allows contact between the raw material gas and the catalyst structure being heated. For example, the catalyst structure may be held at part or across the whole of the hydrocarbon production unit 103. The FT synthesis reaction with the catalyst structure may be carried out using, for example, a fixed bed, a supercritical fixed bed, a slurry bed, or a fluidized bed. In particular, a fixed bed, a supercritical fixed bed, or a slurry bed is preferred.

Regarding the location of the second separation unit 110, FIG. 1 shows an example in which the second separation unit 110 receives supply of the hydrocarbons discharged from the hydrocarbon production unit 103. Alternatively, the second separation unit 110 may be located to receive supply of the hydrocarbons discharged from the first separation unit 111. The production apparatus 100 may also include multiple sets of the second separation unit 110.

### Method for Producing Hydrocarbons

The hydrocarbon production method according to an embodiment of the present invention is a method for producing hydrocarbons including at least one compound selected from lower olefins including propylene and aromatic hydrocarbons having six or more and ten or less carbon atoms, for example, which may be carried out using the production apparatus 100 shown in FIG. 1.

The hydrocarbon production method according to an embodiment of the present invention includes, as main steps, a first supply step S1 and a production step S2.

The first supply step S1 includes supplying, to a catalyst structure, a raw material gas including carbon monoxide and hydrogen. In the production apparatus 100 shown in FIG. 1, the raw material gas is supplied from the first supply unit 101 to the catalyst structure in the catalyst structure holder 104 provided inside the hydrocarbon production unit 103.

The production step S2 includes producing, from carbon monoxide and hydrogen in the raw material gas supplied in the first supply step S1, hydrocarbons including at least one compound selected from lower olefins including propylene and aromatic hydrocarbons having six or more and ten or less carbon atoms while heating the catalyst structure at a first temperature of 150°C or more and less than 300°C or at a second temperature of 350°C or more and less than 550°C. In the production apparatus 100, the hydrocarbons are produced from carbon monoxide and hydrogen in the raw material gas using the catalyst structure being heated at a predetermined temperature in the hydrocarbon production unit 103.

The hydrocarbon production method according to an embodiment may further include an oxygen supply step S11 that includes supplying oxygen to the catalyst structure. Oxygen is supplied from the oxygen supply unit 114 to the catalyst structure in the catalyst structure holder 104. This can prevent a decrease in catalytic activity, which would otherwise be caused by coking of the catalyst structure in the production step S2.

The hydrocarbon production method according to an embodiment may further include a first separation step S12 that includes separating carbon monoxide and hydrogen from the hydrocarbons produced in the production step S2. The first separation unit 111 can separate carbon monoxide and hydrogen from the hydrocarbons produced in the production step S2, so that the hydrocarbons can be produced at a high volume content.

The hydrocarbon production method according to an embodiment may further include a second supply step S13 that includes feeding, to the production step S2, carbon monoxide and hydrogen separated in the first separation step S12. The second supply unit 116 feeds carbon monoxide and hydrogen to the production step S2 so that carbon monoxide and hydrogen are reused for the FT synthesis reaction in the production step S2.

The hydrocarbon production method according to an embodiment may further include a detection step S14 that includes detecting propylene and the aromatic hydrocarbons in the hydrocarbons produced in the production step S2. In the production apparatus 100, the detection unit 115 detects propylene and the aromatic hydrocarbons in the hydrocarbons produced in and discharged from the hydrocarbon production unit 103.

The hydrocarbon production method according to an embodiment may further include a second separation step S15 that includes cooling the hydrocarbons produced in the production step S2 and separating the aromatic hydrocarbons and a gas including the lower olefins. The second separation unit 110 separates the lower olefins and the aromatic hydrocarbons by cooling the hydrocarbons.

The hydrocarbon production method according to an embodiment may further include a third separation step S16 that includes separating water from the aromatic hydrocarbons obtained in the second separation step S15. The third supply unit 112 separates water from the aromatic hydrocarbons obtained in the second separation step S15.

### Configuration of Catalyst structure

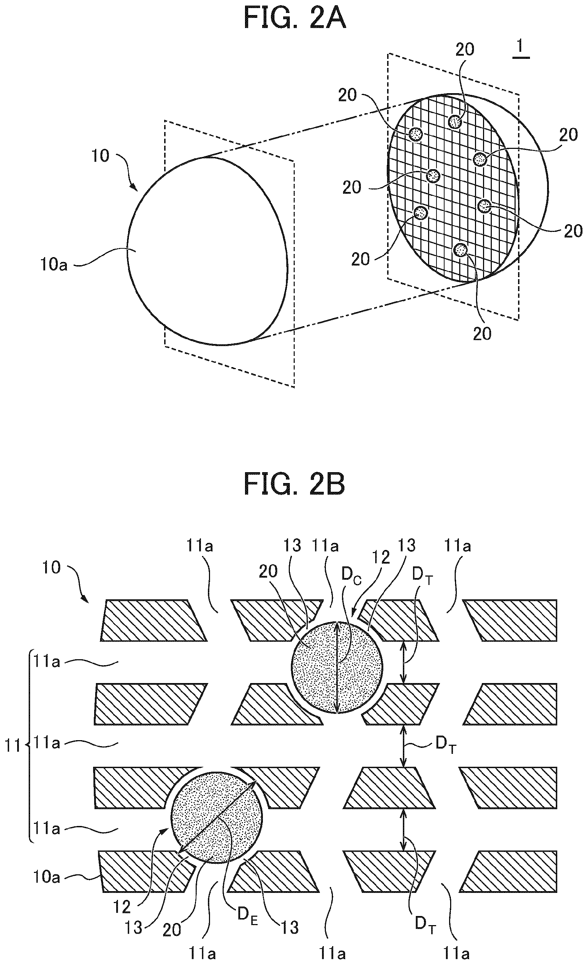

FIGS. 2(a) and 2(b) are views schematically showing the configuration of the catalyst structure 1 held in the catalyst structure holder 104 of the hydrocarbon production apparatus 100 described above, in which FIG. 2(a) is a perspective view (shown partially in transverse cross-sectional view), and FIG. 2(b) is a partially enlarged cross-sectional view. It should be noted that FIGS. 2(a) and 2(b) show only an example of the catalyst structure and the configuration shown in FIGS. 2(a) and 2(b), such as shapes and dimensions, are not intended to limit those of the present invention. The catalyst structure 1 is suitable for use in FT synthesis reaction.

As shown in FIG. 2(a), the catalyst structure 1 includes a support 10 having a porous structure and including a zeolite-type compound; and at least one metal fine particle 20 present in the support 10.

In the catalyst structure 1, multiple metal fine particles 20 are included in the porous structure of the support 10. The metal fine particles 20 are each a catalytic material having catalytic ability (catalytic activity). The metal fine particles will be described in detail later. The metal fine particles 20 may include a metal oxide, a metal alloy, or a composite of a metal oxide and a metal alloy.

As shown in FIG. 2(b), the support 10 has a porous structure and has channels 11 communicating with outside the support 10. The support 10 preferably has multiple pores 11a, which form the channels 11. In the support 10, some of the channels 11 have an average inner diameter of 0.95 nm or less. The metal fine particles 20 are present in channels 11 with an average inner diameter of 0.95 nm or less and preferably held in channels 11 with an average inner diameter of 0.95 nm or less.

In particular, such a configuration increase the selectivity of the FT synthesis reaction for the hydrocarbons. Such a configuration also restrict the movement of the metal fine particles 20 in the support 10 and effectively prevent aggregation of the metal fine particles 20. This results in effective prevention of a decrease in the effective surface area of the metal fine particles 20 and results in long-term retention of the catalytic activity of the metal fine particles 20. In other words, the configuration of the catalyst structure 1 make it possible to prevent a decrease in catalytic activity, which would otherwise be caused by aggregation of the metal fine particles 20, and to prolong the life of the catalyst structure 1. Moreover, thanks to the prolonged life of the catalyst structure 1, the frequency of replacement of the catalyst structure 1 can be reduced, and the amount of the used catalyst structure 1 discarded can be greatly reduced, which leads to resource saving.

In general, when used in a fluid (e.g., heavy oil, reforming gas such as NOₓ), a catalyst structure may receive an external force from the fluid. In such a case, if metal fine particles are only deposited on the outer surface of the support 10, there will be a problem in that, due to the influence of the external force from the fluid, the metal fine particles can easily separate from the outer surface of the support 10. On the other hand, in the catalyst structure 1, the metal fine particles 20 are present at least in channels 11 of the support 10 and thus less likely to separate from the support 10 even when receiving an external force from a fluid. Specifically, when the catalyst structure 1 is placed in a fluid, the fluid flowing into the channels 11 through the pores 11a of the support 10 encounters flow channel resistance (frictional force), so that the velocity of the fluid flowing in the channels 11 would be lower than that of the fluid flowing on the outer surface of the support 10. Due to the influence of such flow channel resistance, the pressure applied from the fluid onto the metal fine particles 20 present in the channels 11 becomes lower than that applied outside the support 10. Therefore, the metal fine particles 20 are effectively prevented from separating from the support 10, and the catalytic activity of the metal fine particles 20 can be stably maintained for a long period of time. The flow channel resistance would be higher when the channel 11 of the support 10 has multiple curves or branches and the interior of the support 10 has a more complicated three-dimensional structure.

The channels 11 preferably have any one of a one-dimensional pore, a two-dimensional pore, and a three-dimensional pore, which are defined by the framework structure of the zeolite-type compound, and preferably have enlarged pore portions 12 different from all of the one-dimensional pore, the two-dimensional pore, and the three-dimensional pore. In this case, the metal fine particles 20 are preferably present at least in the enlarged pore portions 12 and more preferably included at least in the enlarged pore portions 12. The enlarged pore portion 12 also preferably connects multiple pores 11a to one another when the pores 11a form any of the one-dimensional pore, the two-dimensional pore, and the three-dimensional pore. According to this configuration, another channel different from the one-dimensional pore, the two-dimensional pore, or the three-dimensional pore is provided in the support 10 to exert the function of the metal fine particles 20 more effectively. As used herein, the term "one-dimensional pore" or "one-dimensional pores" refers to a tunnel- or cage-shaped pore that forms a one-dimensional channel or refers to multiple tunnel- or cage-shaped pores that form multiple one-dimensional channels. The term "two-dimensional pore" refers to a channel having multiple one-dimensional channels connected two-dimensionally. The term "three-dimensional pore" refers to a channel having multiple one-dimensional channels connected three-dimensionally. According to this configuration, the movement of the metal fine particles 20 is further restricted in the support 10, and separation of the metal fine particles 20 and aggregation of the metal fine particles 20 are more effectively prevented. The state in which the catalytic material 20 is included in the porous structure of the support 10 indicates that the catalytic material 20 is enclosed within the support 10. In this regard, the metal fine particle 20 and the support 10 do not always have to be in direct contact with each other, and the metal fine particle 20 may be indirectly held by the support 10 with an additional material (e.g., a surfactant) provided between the metal fine particle 20 and the support 10.

FIG. 2(b) shows a case in which the metal fine particle 20 is included in the enlarged pore portion 12. Such a configuration is non-limiting, and alternatively, the metal fine particle 20 may be held in the channel 11 while partially protruding out of the enlarged pore portion 12. Alternatively, the metal fine particle 20 may be partially embedded in a portion of the channel 11 other than the enlarged pore portion 12 (e.g., an inner wall portion of the channel 11) or may be held by fixation or the like.

The channel 11 preferably has a three-dimensional structure including a branching or junction portion inside the support 10, and the enlarged pore portion 12 is preferably provided at the branching or junction portion of the channel 11.

The average inner diameter D_{F} of all channels 11 provided in the support 10 may be calculated from the average of the short and long diameters of the pores 11a, which form any of the one-dimensional pore, the two-dimensional pore, and the three-dimensional pore. The average inner diameter D_{F} of the channels 11 is typically 0.10 nm or more and 1.50 nm or less and preferably 0.49 nm or more and 0.95 nm or less. For synthesis of light hydrocarbons by the FT synthesis reaction, carbon monoxide and hydrogen as raw materials for the FT synthesis reaction have molecular sizes of about 0.38 nm and about 0.29 nm, respectively. Therefore, the average inner diameter D_{F} of all channels 11 is preferably 0.40 nm or more and 1.50 nm or less, more preferably 0.49 nm or more and 0.95 nm or less, and even more preferably 0.49 nm or more and 0.70 nm or less. The inner diameter D_{E} of the enlarged pore portion 12 is typically 0.5 nm or more and 50 nm or less, preferably 1.1 nm or more and 40 nm or less, and more preferably 1.1 nm or more and 3.3 nm or less. The inner diameter D_{E} of the enlarged pore portion 12 depends, for example, on the pore size of the precursor material (A) described later and the average particle size D_{C} of the metal fine particles 20 to be included. The inner diameter D_{E} of the enlarged pore portion 12 is such that it is possible to include the metal fine particle 20.

The support 10 includes a zeolite-type compound. Examples of the zeolite-type compound include silicate compounds, such as zeolite (aluminosilicate), cation-exchanged zeolite, and silicalite; zeolite analogue compounds, such as aluminoborates, aluminoarsenates, and germanates; and phosphate-based zeolite analogue materials, such as molybdenum phosphate. Among them, the zeolite-type compound is preferably a silicate compound.

The framework structure of the zeolite-type compound may be selected from FAU type (Y or X type), MTW type, MFI type, FER type (ferrierite), LTA type (A type), MWW type (MCM-22), MOR type (mordenite), LTL type (L type), BEA type (beta type), and so on, and is preferably MFI type, MOR type, or BEA type.

The zeolite-type compound has multiple pores with a diameter depending on its framework structure. For example, MFI type ([010] axis) has a short pore diameter of 0.53 nm (5.30 Å), a long pore diameter of 0.56 nm (5.50 Å), and an average pore diameter (average inner diameter) of about 0.55 nm (5.50 Å). If the resulting product has a size (typically a molecular size) smaller than the inner diameter of the channels 11 in the support 10, which depends on the framework structure of the zeolite-type compound, the resulting product will be restricted from moving through the channels 11. During the hydrocarbon synthesis, the carbon chain grows as the reactive material comes into contact with the metal fine particles in the pores of the zeolite-type compound. In the support 10 including the zeolite-type compound, therefore, some of the channels 11, specifically, channels 11 holding the metal fine particles 20 as a catalytic material should have an average inner diameter D_{T} of 0.95 nm or less. This configuration can control the carbon chain growth based on the pore size of the zeolite-type compound and can suppress the production of heavy hydrocarbons with a relatively large molecular size. When the average inner diameter D_{T} of some channels 11 holding the metal fine particles 20 is controlled to 0.95 nm or less as mentioned above, the catalyst structure can have high selectivity for production of the hydrocarbons, for example, by the Fischer-Tropsch synthesis reaction. Although depending on the framework structure of the zeolite-type compound, the average inner diameter D_{T} preferably has a lower limit of 0.39 nm or more for production of materials having three or more carbon atoms, which are useful as petrochemical raw materials. The pores in the zeolite-type compound are not always circular and may be polygonal in some cases. In such cases, the average inner diameter D_{T} may be calculated, for example, by evenly dividing the sum of the long pore diameters (long axes) and the short pore diameters (short axes) of the pores.

When the average inner diameter D_{T} of some channels holding the metal fine particles is more strictly controlled, the catalyst structure can have higher selectivity for production of light hydrocarbons and in particular can have higher selectivity for production of light hydrocarbons having a molecular size substantially the same as the average inner diameter D_{T}. For example, light hydrocarbons with three carbon atoms have a molecular size of 0.49 nm or more and less than 0.59 nm, light hydrocarbons with four carbon atoms have a molecular size of 0.59 nm or more and less than 0.70 nm, light hydrocarbons with five carbon atoms have a molecular size of 0.70 nm or more and less than 0.79 nm, and light hydrocarbons with six carbon atoms have a molecular size of 0.79 nm or more and less than 0.95 nm. N-Hexene with six carbon atoms has a molecular size of about 0.91 nm, n-pentene with five carbon atoms has a molecular size of about 0.78 nm, n-butene with four carbon atoms has a molecular size of about 0.65 nm, propylene with three carbon atoms has a molecular size of about 0.52 nm, and ethylene with two carbon atoms has a molecular size of about 0.39 nm. Therefore, some of the channels 11 preferably have an average inner diameter D_{T} of 0.75 nm or less in order to increase the selectivity for production of hydrocarbons with four or less carbon atoms, preferably have an average inner diameter D_{T} of less than 0.75 nm and more preferably 0.55 nm or more and less than 0.75 nm in order to increase the selectivity for production of hydrocarbons with four carbon atoms, such as n-butene, and more preferably have an average inner diameter D_{T} of 0.63 nm or more and less than 0.75 nm for higher selectivity for production of hydrocarbons with four carbon atoms than for production of hydrocarbons with three carbon atoms and for more selective collection of n-butene. In order to increase the selectivity for production of hydrocarbons with three carbon atoms, such as propylene, some of the channels 11 preferably have an average inner diameter D_{T} of less than 0.68 nm and more preferably more than 0.39 nm and less than 0.68 nm. The selectivity for production of light hydrocarbons is sometimes affected not only by the pore size of the zeolite-type compound but also by the framework structure of the zeolite-type compound, the motion of the produced light hydrocarbon molecules, and other factors. For example, when the framework structure of the zeolite-type compound is MFI type, the selectivity for production of such olefins as propylene and butene (n-butene and isobutene) tends to be high. Therefore, even for production of light hydrocarbons with a molecular size larger than the average inner diameter D_{T}, the selectivity can be increased using the influence of these factors.

The metal fine particles 20, which may be primary particles or secondary particles resulting from aggregation of primary particles, preferably have an average particle size D_{C} larger than the average inner diameter D_{F} of all channels 11 and equal to or smaller than the inner diameter D_{E} of the enlarged pore portion 12 (D_{F} < D_{C} ≤ D_{E}). The metal fine particles 20 with such a configuration are preferably present in the enlarged pore portions 12 in the channels 11, so that the movement of the metal fine particles 20 is restricted in the support 10. Therefore, even when an external force is applied from a fluid to the metal fine particles 20, the movement of the metal fine particles 20 is suppressed in the support 10, so that the metal fine particles 20 dispersed in channels 11 of the support 10 and respectively present in the enlarged pore portions 12 are effectively prevented from coming into contact with one another.

The metal fine particles 20, which may be either of primary particles and secondary particles, preferably have an average particle size D_{C} of 0.08 nm or more. The ratio (D_{C}/D_{F}) of the average particle size D_{C} of the metal fine particles 20 to the average inner diameter D_{F} of all channels 11 is preferably 0.05 or more and 300 or less, more preferably 0.1 or more and 30 or less, even more preferably 1.1 or more and 30 or less, and further more preferably 1.4 or more and 3.6 or less. The content of the metal element (M) of the metal fine particles 20 is preferably 0.5 mass% or more and 7.6 mass% or less, more preferably 0.5 mass% or more and 6.9 mass% or less, even more preferably 0.5 mass% or more and 2.5 mass% or less, and most preferably 0.5 mass% or more and 1.5 mass% or less with respect to the mass of the catalyst structure 1. For example, when the metal element (M) is Co, the content (mass%) of the Co element is expressed by {(the mass of Co element)/(the mass of all elements in the catalyst structure 1)} × 100.

The metal fine particles only have to include metal that is intact and not oxidized. For example, the metal fine particles may include a single metal or a mixture of two or more metals. When used herein to indicate the component (material) of the metal fine particles, the term "metal" is a generic term for a metallic material including one or more metal elements, which is intended to include an elementary metal including a single metal element (M) and an alloy including two or more metal elements (M). In some environments where the catalyst structure is used, an oxide of the metal element can be reduced to metal fine particles. In such cases, the metal oxide may be deemed to be equivalent to metal fine particles.

The metal fine particles may include a first metal or first and second metals. The first metal may be at least one selected from the group consisting of ruthenium (Ru), nickel (Ni), iron (Fe), and cobalt (Co). The second metal may be at least one different from the first metal and selected from the group consisting of platinum (Pt), palladium (Pd), gold (Au), silver (Ag), copper (Cu), ruthenium (Ru), iridium (Ir), rhodium (Rh), osmium (Os), zirconium (Zr), and manganese (Mn). When the second metal is Ru, the first metal may be at least one selected from the group consisting of Ni, Fe, and Co. In particular, to be useful for the FT synthesis reaction, the metal fine particles preferably include at least one of Co, Fe, Ni, and Ru. For higher catalytic activity for the FT synthesis reaction, the metal fine particles preferably include the second metal in addition to the first metal selected from Co, Fe, Ni, and Ru.

The ratio (Si/M atomic ratio) of the number of silicon (Si) atoms in the support 10 to the number of the metal element (M) in the metal fine particles 20 is preferably 10 or more and 1000 or less, more preferably 50 or more and 200 or less, and even more preferably 100 or more and 200 or less. If the ratio is higher than 1000, the metal fine particles may have low catalytic activity or fail to act sufficiently as a catalytic material. If the ratio is lower than 10, the content of the metal fine particles 20 may be so high as to tend to reduce the strength of the support 10. It should be noted that, in this context, the term "metal fine particles 20" refers to fine particles present or held inside the support 10 and is not intended to include metal fine particles deposited on the outer surface of the support 10.

### Function of Catalyst structure

As mentioned above, the catalyst structure 1 includes a support 10 of a porous structure and at least one metal fine particle 20 in the support 10. When brought into contact with a fluid, the catalyst structure 1 exerts the catalytic ability of the metal fine particles 20 in the support 10. Specifically, when coming into contact with the outer surface 10a of the catalyst structure 1, the fluid is allowed to flow into the interior of the support 10 through a pore 11a at the outer surface 10a, guided into the channels 11, and allowed to pass through the channels 11 and to flow out of the catalyst structure 1 through another pore 11a. The metal fine particles 20 in some of the channels 11, which have an average inner diameter of 0.95 nm or less, cause a catalytic reaction when coming into contact with the fluid passing through the channels 11. The catalyst structure 1 also has a molecular sieving ability since the support has a porous structure.

First, the molecular sieving ability of the catalyst structure 1 will be described with reference to an example in which the fluid includes carbon monoxide and hydrogen. The catalyst structure may be brought into contact with carbon monoxide and hydrogen sequentially or simultaneously. Compound molecules (e.g., carbon monoxide and hydrogen) having a size equal to or smaller than the diameter of the pore 11a, in other words, equal to or smaller than the inner diameter of the channel 11, can enter the support 10. On the other hand, gas component molecules having a size exceeding the diameter of the pore 11a cannot enter the support 10. Accordingly, among multiple compounds in the fluid, some compounds not capable of entering the support 10 are restricted from reacting, and some other compounds capable of entering the support 10 are allowed to react.

Among compounds produced by reactions in the support 10, only compounds having a molecular size not exceeding the diameter of the pore 11a can pass through the pore 11a to outside the support 10 to obtain a reaction product. On the other hand, some compounds are not capable of passing through the pore 11a to outside the support 10. If such compounds are converted into compounds having a molecular size that allows exit from the support 10, the compounds can go outside the support 10. Even with a size larger than the diameter of the pore 11a, some produced compound molecules in motion can go outside the support 10 while expanding and contracting. Therefore, a specific reaction product can be selectively obtained using the catalyst structure 1 in which the diameter of pores 11a is controlled, specifically, the diameter of pores 11a holding the metal fine particles is controlled. In this embodiment, specifically, carbon monoxide and hydrogen react to yield hydrocarbons as reaction products, which include at least one compound selected from lower olefins including propylene and aromatic hydrocarbons having six or more and ten or less carbon atoms.

In the catalyst structure 1, the metal fine particles 20 are included in the enlarged pore portions 12 of the channels 11. When the average particle size D_{C} of the metal fine particles is larger than the average inner diameter D_{T} of some of the channels 11 and smaller than the inner diameter D_{E} of the enlarged pore portion 12 (D_{T} < D_{C} < D_{E}), a small channel 13 is provided between the metal fine particle and the enlarged pore portion 12. In this case, the fluid entering the small channel 13 comes into contact with the metal fine particle. Each metal fine particle included in the enlarged pore portion 12 is restricted from moving in the support 10. Thus, the metal fine particles are prevented from aggregating in the support 10. As a result, a large contact area can be stably maintained between the metal fine particles and the fluid.

Specifically, when a molecule (a material to be reformed) entering the channel 11 comes into contact with the metal fine particles 20, a catalytic reaction occurs to reform the molecule. For example, lower olefins and the aromatic hydrocarbons can be selectively produced from a mixed gas composed mainly of hydrogen and carbon monoxide as raw materials by using the catalyst structure 1 with controlled average inner diameter D_{T} of some of the channels 11. The FT synthesis reaction of hydrogen and caron monoxide as main components, although carried out, for example, at the first or second temperature, is less affected by the heating, since the metal fine particles 20 are incorporated in the support 10. In particular, the metal fine particles 20 in the enlarged pore portions 12 are more restricted from moving in the support 10, so that the metal fine particles 20 are more effectively prevented from aggregating (sintering). As a result, a decrease in catalytic activity is more effectively prevented, which allows the catalyst structure 1 to have a longer life. Even if the catalyst structure 1 has reduced catalytic activity after long-term use, the metal fine particles 20, which are not bonded to the support 10, can be easily activated (reduced).

### Method of Producing Catalyst structure

Hereinafter, an example of a method of producing the catalyst structure will be described with reference to an example in which the metal fine particles in the support include an oxidation-resistant metal species.

### Step S1-1: Preparation Step

First, a precursor material (A) is prepared, which is for forming a support having a porous structure and including a zeolite-type compound. The precursor material (A) is preferably an ordered mesoporous material, and may be appropriately selected depending on the type (composition) of the zeolite-type compound for forming the support of the catalyst structure.

When a silicate compound is used as the zeolite-type compound for forming the support of the catalyst structure, the ordered mesoporous material is preferably a compound having an Si-O framework having pores with a diameter of 1 nm or more and 50 nm or less uniformly and regularly developed in a one-dimensional pattern, a two-dimensional pattern, or a three-dimensional pattern. A variety of synthetic products can be obtained as such ordered mesoporous materials depending on the synthesis conditions. Examples of such synthetic products include SBA-1, SBA-15, SBA-16, KIT-6, FSM-16, and MCM-41. In particular, MCM-41 is preferred. For reference, SBA-1 has a pore size of 10 nm or more and 30 nm or less, SBA-15 has a pore size of 6 nm or more and 10 nm or less, SBA-16 has a pore size of 6 nm, KIT-6 has a pore size of 9 nm, FSM-16 has a pore size of 3 nm or more and 5 nm or less, and MCM-41 has a pore size of 1 nm or more and 10 nm or less. Examples of such an ordered mesoporous material include mesoporous silica, mesoporous aluminosilicate, and mesoporous metallosilicate.

The precursor material (A) may be any of a commercially available product and a synthetic product. The precursor material (A) may be synthesized using a known method for synthesizing an ordered mesoporous material. For example, a mixture solution is prepared, which contains a raw material containing elements for forming the precursor material (A) and a template agent for directing the structure of the precursor material (A). Optionally after being subjected to pH adjustment, the mixture solution is subjected to hydrothermal treatment (hydrothermal synthesis). Subsequently, the precipitate (product) resulting from the hydrothermal treatment is collected (e.g., filtered off), washed and dried if necessary, and then calcinated to obtain a precursor material (A) as a powdery ordered mesoporous material. In this process, the solvent for the mixture solution may be, for example, water, an organic solvent such as an alcohol, or a mixed solvent thereof. The raw material may be selected depending on the type of the support. Examples of the raw material include silica agents, such as tetraethoxysilane (TEOS), fumed silica, and quartz sand. The template agent may be any of various surfactants and block copolymers. The template agent is preferably selected depending on the type of the ordered mesoporous material to be synthesized. For example, the template agent for use in forming MCM-41 is preferably a surfactant such as hexadecyltrimethylammonium bromide. The hydrothermal treatment may be performed, for example, in a closed vessel under conditions at 80°C or more and 800°C or less and 0 kPa or more and 2000 kPa or less for 5 hours or more and 240 hours or less. The calcining treatment may be performed, for example, in the air under conditions at 350°C or more and 850°C or less for 2 hour or more and 30 hours or less.

### Step S1-2: Impregnation Step

Next, the prepared precursor material (A) is impregnated with a metal-containing solution to form a precursor material (B) .

The metal-containing solution may be any solution containing a metal component (e.g., metal ions) corresponding to the metal element (M) for forming the metal fine particles for the catalyst structure. For example, the metal-containing solution may be prepared by dissolving, in a solvent, a metal salt containing the metal element (M). Examples of such a metal salt include chlorides, hydroxides, oxides, sulfates, and nitrates, among which nitrates are preferred. The solvent may be, for example, water, an organic solvent such as an alcohol, or a mixture solvent thereof.

Any method may be used to impregnate the precursor material (A) with the metal-containing solution. For example, before the calcining step described later, the impregnation is preferably performed by adding the metal-containing solution little by little in multiple portions to the powdery precursor material (A) being stirred. In order to allow the metal-containing solution to more easily enter the inner pores of the precursor material (A), a surfactant is preferably added as an additive to the precursor material (A) in advance before the addition of the metal-containing solution. Such an additive can act to cover the outer surface of the precursor material (A) and thus to inhibit the deposition of the metal-containing solution on the outer surface of the precursor material (A), so that the metal-containing solution added subsequently could easily enter the pores of the precursor material (A).

Examples of such an additive include nonionic surfactants, such as polyoxyethylene oleyl ether, polyoxyethylene alkyl ether, and polyoxyethylene alkyl phenyl ether. These surfactants have a large molecular size and thus cannot enter the inner pores of the precursor material (A), which suggests that they will not adhere to the interior of the pores and will not hinder the entry of the metal-containing solution into the pores. A method of adding the nonionic surfactant preferably includes, for example, adding 50 mass% or more and 500 mass% or less of the nonionic surfactant to the precursor material (A) before the calcining step described later. If the amount of the nonionic surfactant added to the precursor material (A) is less than 50 mass%, the inhibiting effect may be difficult to achieve, and if the amount of the nonionic surfactant is more than 500 mass% relative to the amount of the precursor material (A), the solution may have undesirably high viscosity. Therefore, the amount of the nonionic surfactant added to the precursor material (A) should be set to a value within the above range.

The amount of the metal element (M) in the metal-containing solution, with which the precursor material (A) is to be impregnated (in other words, the amount of the metal element (M) to be incorporated into the precursor material (B)) is preferably taken into account when the amount of the metal-containing solution added to the precursor material (A) is appropriately adjusted. For example, before the calcining step described later, the amount of the metal-containing solution added to the precursor material (A) is preferably adjusted such that the ratio (Si/M atomic ratio) of the number of silicon (Si) atoms in the precursor material (A) to the number of the metal element (M) in the metal-containing solution is set to 10 or more and 1000 or less, more preferably 50 or more and 200 or less, and even more preferably 100 or more and 200 or less. For example, when a surfactant is added as an additive to the precursor material (A) before the addition of the metal-containing solution to the precursor material (A), the amount of the metal-containing solution added to the precursor material (A) may be adjusted such that the calculated Si/M atomic ratio can be 50 or more and 200 or less. In such a case, the content of the metal element (M) of the metal fine particles can be adjusted to 0.5 mass% or more and 7.6 mass% or less based on the mass of the catalyst structure. In the precursor material (B), the content of the metal element (M) in the inner porous portion is approximately proportional to the amount of the metal-containing solution added to the precursor material (A) as long as the metal concentration of the metal-containing solution, the presence or absence of the additive, and other conditions such as temperature and pressure remain constant. The amount of the metal element (M) in the precursor material (B) is also proportional to the amount of the metal element in the metal fine particles in the support of the catalyst structure. Accordingly, when the amount of the metal-containing solution added to the precursor material (A) is controlled within the above range, the inner pores of the precursor material (A) can be impregnated with a sufficient amount of the metal-containing solution, which makes it possible to adjust the content of the metal fine particles in the support of the catalyst structure.

After the precursor material (A) is impregnated with the metal-containing solution, washing treatment may be performed if necessary. The washing liquid used may be water, an organic solvent such as an alcohol, or a mixed solution thereof. Drying treatment is also preferably performed after the impregnation of the precursor material (A) with the metal-containing solution and optionally after the washing treatment. The drying treatment may include natural drying overnight or so or drying at a high temperature of 150°C or less. The drying is preferably performed thoroughly because the framework structure of the precursor material (A) for the ordered mesoporous material may collapse if the calcining treatment described later is performed while a large amount of water derived from the metal-containing solution or the washing liquid remains in the precursor material (A).

### Step S1-3: Calcining Step

Next, the precursor material (B) is calcinated to form a precursor material (C). The precursor material (B) is a product obtained through impregnating, with the metal-containing solution, the precursor material (A) for forming the support having a porous structure and including the zeolite-type compound.

The calcining is preferably carried out, for example, in the air under conditions at 350°C or more and 850°C or less for 2 hours or more and 30 hours or less. Such calcining treatment allows the growth of crystals of the metal component deposited by the impregnation in the pores for the ordered mesoporous material, so that the metal fine particles are formed in the pores.

### Step S1-4: Hydrothermal Treatment Step

Then, the precursor material (C), obtained through calcining the precursor material (B), and a structure-directing agent are mixed to form a mixture solution, which is hydrothermally treated to form a catalyst structure.

The structure-directing agent is a template agent for directing the framework structure of the support of the catalyst structure. The structure-directing agent may be, for example, a surfactant. The structure-directing agent is preferably selected depending on the framework structure of the support in the catalyst structure, and preferred examples thereof include a surfactant such as tetramethylammonium bromide (TMABr), tetraethylammonium bromide (TEABr), tetrapropylammonium bromide (TPABr), tetraethylammonium hydroxide (TEAOH).

The precursor material (C) and the structure-directing agent may be mixed during or before the hydrothermal treatment step. Any method may be used to prepare the mixture solution. The precursor material (C), the structure-directing agent, and the solvent may be mixed at the same time, or the precursor material (C) and the structure-directing agent may be separately dispersed into individual solvents, and then the resulting dispersion solutions may be mixed. The solvent may be, for example, water, an organic solvent such as an alcohol, or a mixed solvent thereof. Before the hydrothermal treatment, the mixture solution is preferably subjected to pH adjustment using an acid or a base.

The hydrothermal treatment may be carried out using a known method, for example, which is preferably performed in a closed vessel under conditions at 80°C or more and 800°C or less and 0 kPa or more and 2000 kPa or less for 5 hours or more and 240 hours or less. The hydrothermal treatment is also preferably performed in a basic atmosphere. Although the reaction mechanism is not necessarily clear, the hydrothermal treatment using the precursor material (C) as a starting material can gradually destroy the framework structure of the precursor material (C) for the ordered mesoporous material but can form a new framework structure (porous structure) for the support of the catalyst structure due to the action of the structure-directing agent while the position of the metal fine particles in the pores of the precursor material (C) substantially remains. The resulting catalyst structure includes a support of a porous structure and metal fine particles in the support, in which the support has channels connecting multiple pores derived from the porous structure, and at least some of the metal fine particles are located in channels of the support. In the embodiment, the hydrothermal treatment step includes preparing a solution of a mixture of the precursor material (C) and the structure-directing agent and hydrothermally treating the precursor material (C) in the mixture solution. This step is non-limiting, and alternatively, the precursor material (C) may be hydrothermally treated without being mixed with the structure-directing agent.

Preferably, the precipitate (catalyst structure) resulting from the hydrothermal treatment is collected (e.g., filtered off) and then optionally washed, dried, and calcinated. The washing liquid may be water, an organic solvent such as an alcohol, or a mixed solution thereof. The drying may include natural drying overnight or so or drying at a high temperature of 150°C or less. The drying is preferably performed thoroughly because the framework structure for the support of the catalyst structure may collapse if the calcining treatment is performed while a large amount of water remains in the precipitate. The calcining treatment may be performed, for example, in the air under conditions at 350°C or more and 850°C or less for 2 hours or more and 30 hours or less. During such calcining treatment, the structure-directing agent is burned away from the catalyst structure. Depending on the intended use, the catalyst structure may be used as it is without undergoing the calcining treatment of the collected precipitate. For example, when the catalyst structure is used in a high-temperature oxidative atmosphere environment, the structure-directing agent will be burned away by being exposed to the usage environment for a certain period of time. In such a case, the resulting catalyst structure can used without any modification since it is substantially the same as that obtained after the calcining treatment.

The production method described above is an exemplary method for the case where an oxidation-resistant metal species (e.g., noble metal) is used as the metal element (M) to form the metal-containing solution with which the precursor material (A) is to be impregnated.

An easily oxidizable metal species (e.g., Fe, Co, Ni) may also be used as the metal element (M) to form the metal-containing solution with which the precursor material (A) is to be impregnated. In such a case, after the hydrothermal treatment, the hydrothermally treated precursor material (C) is preferably subjected to reduction treatment. When the metal-containing solution contains such an easily oxidizable metal species as the metal element (M), the metal component can be oxidized by heating in the steps (S1-3 and S1-4) after the impregnation step (S1-2). Accordingly, the support formed in the hydrothermal treatment step (S1-4) may contain metal oxide fine particles. In order to obtain a catalyst structure including a support containing metal fine particles, therefore, the hydrothermal treatment is preferably followed by calcining treatment of the collected precipitate and then preferably followed by reduction treatment in a reducing gas atmosphere, such as hydrogen gas. The reduction treatment reduces the metal oxide fine particles in the support to form fine particles of the metal element (M) (metal fine particles). As a result, a catalyst structure is obtained including a support containing metal fine particles. It should be noted that such reduction treatment may be performed as needed. For example, if the catalyst structure is used in a reducing atmosphere environment, the metal oxide fine particles can be reduced by being exposed to the usage environment for a certain period of time. In such a case, the resulting catalyst structure can be used without any modification since it is substantially the same as that obtained after the reduction treatment.

### Modifications of Catalyst structure

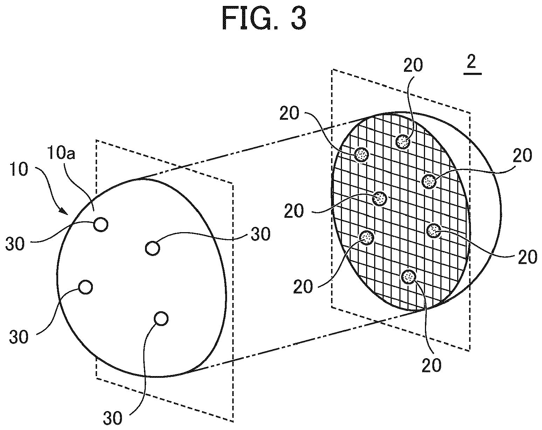

FIG. 3 is a schematic view showing a modification of the catalyst structure 1 of FIGS. 2(a) and 2(b), which will be referred to as a catalyst structure 2. The catalyst structure 1 shown in FIGS. 2(a) and 2(b) includes the support 10 and the metal fine particles 20 in the support 10. Such a structure is non-limiting, and, as shown in FIG. 3, for example, a catalyst structure 2 may be provided, which further includes at least one additional metal fine particle 30 held on an outer surface 10a of the support 10 in addition to the metal fine particles 20 in the support 10.

The metal fine particles 30 are materials that exert one or more types of catalytic abilities. The catalytic ability of the additional metal fine particles 30 may be the same as or different from that of the metal fine particles 20. The metal fine particles 30 having the same catalytic ability as that of the metal fine particles 20 may be made of the same material as that of the metal fine particles 20 or may be made of a material different from that of the metal fine particles 20. According to this configuration, the catalyst structure 2 can have an increased catalytic material content, which further enhances the catalytic activity of the catalytic material.

In this case, the content of the metal fine particles 20 in the support 10 is preferably higher than the content of the additional metal fine particles 30 held on the outer surface 10a of the support 10. In such a case, the catalytic ability of the metal fine particles 20 held inside the support 10 can be dominant, and the catalytic material can stably exhibit its catalytic ability.

While a hydrocarbon production apparatus and a hydrocarbon production method according to embodiments of the present invention have been described, it will be understood that the embodiments are not intended to limit the present invention and may be altered or modified in various ways based on the technical idea of the present invention.

### EXAMPLES

### Example 1-1

First, catalyst structure E1-1 shown in Table 1 was used. Catalyst structure E1-1 included a support and Co present in the support and held on the outer surface of the support. Catalyst structure E1-1 was placed in the interior of a hydrocarbon production unit. Subsequently, while catalyst structure E1-1 was heated at 250°C or more and 500°C or less, carbon monoxide and hydrogen in a volume ratio of 1:2 were supplied (GHSV = 3000 h⁻¹) to the hydrocarbon production unit, and the presence or absence of lower olefins and aromatic hydrocarbons in the product discharged from the hydrocarbon production unit was determined using a detector. As a result, the detector detected olefins having four or less carbon atoms, including propylene, and aromatic hydrocarbons having six or more and ten or less carbon atoms, which indicated that the hydrocarbon production unit successfully produced desired hydrocarbons.

### Evaluation

Next, at each heating temperature, an evaluation was made on each of the degree of selectivity for production of lower olefins and that for production of aromatic hydrocarbons.

### (1) Degree of Selectivity for Production of Lower Olefins

The degree of selectivity for production of lower olefins (olefins having four or less carbon atoms) was defined as the content of the lower olefins in the hydrocarbons in the reaction product. A degree of selectivity of 10% or more was evaluated as good (o), and a degree of selectivity of less than 10% was evaluated as poor (×).

### (2) Degree of Selectivity for Production of Aromatic Hydrocarbons

The degree of selectivity for production of the aromatic hydrocarbons (having six or more and ten or less carbon atoms) was defined as the content of the aromatic hydrocarbons in the hydrocarbons in the reaction product. A degree of selectivity of 5% or more was evaluated as good (o), a degree of selectivity of 1% or more and less than 5% as fair (Δ), and a degree of selectivity of less than 1% as poor (×).

### Comparative Example 1-1

Comparative Example 1-1 was carried out in the same manner as Example 1-1 except that catalyst structure C1-1 shown in Table 1 was used instead of catalyst structure E1-1. The evaluation was then made in the same manner as for Example 1-1. Catalyst structure C1-1 included a support having a porous structure and including the zeolite-type compound; and Co held on the outer surface of the support, in which Co was held only on the outer surface of the support and not located inside the support.

Table 1 shows the conditions for the pretreatment of the catalyst structures, the conditions for the reaction in the hydrocarbon production unit, and the evaluation results.

**[Table 1]**

| | | Example | Comparative Example |
|---|---|---|---|
| | | 1-1 | 1-1 |
| | | Catalyst structure E1-1 | Catalyst structure C1-1 |
| Catalyst structure | Support | Zeolite-type compound | Zeolite-type compound |
| | Metal fine particle | Co | Co |
| | Metal fine particle content (mass%) | 1% | 1% |
| | Location of metal fine particle | Inside and outer surface of support | Outer surface of support |
| | Pretreatment conditions (reduction conditions) | 500°C/1h/H₂ | |
| Reaction conditions | Synthesis gas | Carbon monoxide : hydrogen = 1 : 2 (volume ratio) | |
| | Reaction temperature (°C) | 250° C or more and 500° C or less | |
| Evaluation | Lower olefin selectivity (250°C) | ○ | × |
| | Lower olefin selectivity (300°C) | ○ | × |
| | Lower olefin selectivity (400°C) | ○ | × |
| | Lower olefin selectivity (500°C) | ○ | × |
| | Aromatic hydrocarbon selectivity (250°C) | × | × |
| | Aromatic hydrocarbon selectivity (300°C) | × | × |
| | Aromatic hydrocarbon selectivity (400°C) | Δ | × |
| | Aromatic hydrocarbon selectivity (500°C) | ○ | × |

The results in Table 1 indicate that, in the production apparatus of Example 1-1, desired hydrocarbons were efficiently produced when the catalyst structure was heated at a predetermined temperature. The results suggest that, when catalyst structure E1-1 is heated at a first temperature of 150°C or more and less than 300°C, lower olefins including propylene can be produced from carbon monoxide and hydrogen in the raw material gas without production of aromatic hydrocarbons having six or more and ten or less carbon atoms, so that there is no need for the step of removing the aromatic hydrocarbons from the reaction product obtained using catalyst structure E1-1. The results also suggest that, when catalyst structure E1-1 is heated at a second temperature of 350°C or more and less than 550°C, aromatic hydrocarbons having six or more and ten or less carbon atoms as well as lower olefins including propylene can be efficiently produced from carbon monoxide and hydrogen in the raw material gas.

### EXPLANATION OF REFERENCE NUMERALS

- 100:: Hydrocarbon production apparatus
- 101:: First supply unit
- 102a:: Carbon monoxide supply unit
- 102b:: Hydrogen supply unit
- 103:: Hydrocarbon production unit
- 104:: Catalyst structure holder
- 110:: Second separation unit
- 111:: First separation unit
- 112:: Third separation unit
- 114:: Oxygen supply unit
- 115:: Detection unit
- 116:: Second supply unit
- 1, 2:: Catalyst structure
- 10:: Support
- 10a:: Outer surface
- 11:: Channel
- 11a:: Pore
- 12:: Enlarged pore portion
- 13:: Small channel
- 20, 30:: Metal fine particle

## Claims

1. An apparatus for producing hydrocarbons including at least one compound selected from lower olefins including propylene and aromatic hydrocarbons having six or more and ten or less carbon atoms, the apparatus comprising:
a first supply unit that supplies a raw material gas comprising carbon monoxide and hydrogen; and
a hydrocarbon production unit that comprises a catalyst structure, receives supply of the raw material gas from the first supply unit, and produces, from carbon monoxide and hydrogen in the raw material gas, hydrocarbons including at least one compound selected from lower olefins including propylene and aromatic hydrocarbons having six or more and ten or less carbon atoms while heating the catalyst structure at a first temperature of 150°C or more and less than 300°C or at a second temperature of 350°C or more and less than 550°C, wherein
the catalyst structure includes supports each having a porous structure and including a zeolite-type compound, and at least one metal fine particle present in the supports,
the supports have channels communicating with outside the supports, and
some of the channels have an average inner diameter of 0.95 nm or less.

2. The apparatus for producing hydrocarbons according to claim 1, wherein the aromatic hydrocarbons include at least one of a polycyclic aromatic hydrocarbon and a compound represented by formula (1): 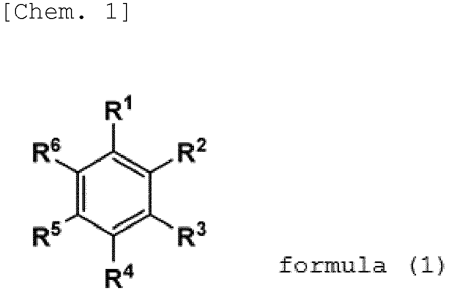 wherein R¹, R², R³, R⁴, R⁵, and R⁶ are each independently a hydrogen atom or an alkyl group having one or more and four or less carbon atoms.

3. The apparatus for producing hydrocarbons according to claim 2, wherein the compound represented by formula (1) includes one or more compounds selected from the group consisting of benzene, toluene, and butylbenzene.

4. The apparatus for producing hydrocarbons according to claim 2, wherein the polycyclic aromatic hydrocarbon includes at least one of azulene and naphthalene.

5. The apparatus for producing hydrocarbons according to any one of claims 1 to 4, further comprising an oxygen supply unit that supplies oxygen to the hydrocarbon production unit.

6. The apparatus for producing hydrocarbons according to claim 5, wherein the oxygen supply unit supplies, to the hydrocarbon production unit, oxygen at a concentration below an explosive limit.

7. The apparatus for producing hydrocarbons according to any one of claims 1 to 6, wherein
the metal fine particle comprises at least one first metal selected from the group consisting of ruthenium (Ru), nickel (Ni), iron (Fe), and cobalt (Co) or
comprises at least one first metal selected from the group consisting of ruthenium (Ru), nickel (Ni), iron (Fe), and cobalt (Co) and at least one second metal being different from the first metal and selected from the group consisting of platinum (Pt), palladium (Pd), gold (Au), silver (Ag), copper (Cu), ruthenium (Ru), iridium (Ir), rhodium (Rh), osmium (Os), zirconium (Zr), and manganese (Mn).

8. The apparatus for producing hydrocarbons according to any one of claims 1 to 7, wherein some of the channels have an average inner diameter of 0.39 nm or more and 0.75 nm or less.

9. The apparatus for producing hydrocarbons according to any one of claims 1 to 8, wherein
the channels have any one of a one-dimensional pore, a two-dimensional pore, and a three-dimensional pore of a framework structure of the zeolite-type compound, and have an enlarged pore portions different from the one-dimensional pore, the two-dimensional pore, and the three-dimensional pore, and
the metal fine particle is present at least in the enlarged pore portion.

10. The apparatus for producing hydrocarbons according to any one of claims 1 to 9, further comprising a first separation unit that separates hydrogen, carbon monoxide, and the hydrocarbons discharged from the hydrocarbon production unit.

11. The apparatus for producing hydrocarbons according to claim 10, further comprising a second supply unit that supplies, to the hydrocarbon production unit, carbon monoxide and hydrogen separated by the first separation unit.

12. The apparatus for producing hydrocarbons according to any one of claims 1 to 11, further comprising a detection unit that detects the propylene and the aromatic hydrocarbons in the hydrocarbons discharged from the hydrocarbon production unit.

13. The apparatus for producing hydrocarbons according to any one of claims 1 to 12, further comprising a second separation unit that cools the hydrocarbons discharged from the hydrocarbon production unit and separates the lower olefins and the aromatic hydrocarbons.

14. A method for producing hydrocarbons including at least one compound selected from lower olefins including propylene and aromatic hydrocarbons having six or more and ten or less carbon atoms, the method comprising:
a step S1 that comprises supplying, to a catalyst structure, a raw material gas comprising carbon monoxide and hydrogen; and
a step S2 that comprises producing, from carbon monoxide and hydrogen in the raw material gas, hydrocarbons including at least one compound selected from lower olefins including propylene and aromatic hydrocarbons having six or more and ten or less carbon atoms while heating the catalyst structure at a first temperature of 150°C or more and less than 300°C or at a second temperature of 350°C or more and less than 550°C, wherein
the catalyst structure includes supports each having a porous structure and including a zeolite-type compound, and at least one metal fine particle present in the supports,
the supports have channels communicating with outside the supports, and
some of the channels have an average inner diameter of 0.95 nm or less.
